# FASCICULE DE BREVET EUROPEEN

(11) **EP 0 787 707 B1**
(45) Date de publication et mention de la délivrance du brevet: **18.09.2002**
(21) Numéro de dépôt: 97400119.0
(22) Date de dépôt: 21.01.1997
(51) Int. Cl.: C07C 17/278, C07C 17/20, C07C 19/08

(54) **Préparation du 1,1,1,3,3-pentachlorobutane et du 1,1,1,3,3-pentafluorobutane**
Herstellung von 1,1,1,3,3-Pentachlorbutan und von 1,1,1,3,3-Pentafluorbutan
Preparation of 1,1,1,3,3-pentachlorobutane and of 1,1,1,3,3-pentafluorobutane

(30) Priorité: 01.02.1996 FR 9601214
(43) Date de publication de la demande: 06.08.1997
(73) Titulaire: Atofina, 92800 Puteaux (FR)
(72) Inventeur: Bertocchio, René, 69390 Vourles par Vernaison (FR); Lantz, André, 69390 Vernaison (FR); Wendlinger, Laurent, 69230 Saint Genis Laval (FR)
(74) Mandataire: Leboulenger, Jean

(56) Documents cités:
- EP-A- 0 699 649
- WO-A-95/04021
- US-A- 3 862 978
- IZV. AKAD. NAUK SSSR, SER. KHIM. (IASKA6,00023353);79; (8); PP.1903-5, INST. ELEMENTOORG. SOEDIN.;MOSCOW; USSR, XP002016172 FREIDLINA R K ET AL: "Telomerization of 2-chloropropene by carbon tetrachloride" & BULLETIN OF THE ACADEMY OF SCIENCES OF THE USSR DIVISION OF CHEM ICAL SCIENCE., vol. 28, no. 7, 1979, NEW YORK US, pages 1766-1769,
- REACT. KINET. CATAL. LETT. (RKCLAU,03044122);91; VOL.44 (2); PP.415-19, CZECH. ACAD. SCI.;INST. CHEM. PROCESS FUNDAM.; PRAGUE; 165 02; CZECH. (CS), XP000605752 KOTORA M ET AL: "Selective additions of polyhalogenated compounds to chloro-substituted ethenes catalyzed by a copper complex"
- Journal of Molecular Catalysis 77, (1992) 51-60

## Description

La présente invention concerne le domaine des hydrocarbures halogénés et a plus particulièrement pour objet la préparation du 1,1,1,3,3-pentachlorobutane et sa fluoration en 1,1,1,3,3-pentafluorobutane.

Avec un point d'ébullition de 40°C, le 1,1,1,3,3-pentafluorobutane (connu sous la désignation F365 mfc) est un substitut potentiel des chlorofluoroalcanes liquides bannis par le protocole de Montréal, tout particulièrement le fluorotrichlorométhane (F11 ; Eb. = 27°C) et le trichlorotrifluoroéthane (F113 ; Eb. = 47°C).

Les procédés permettant de préparer le F365 mfc sont peu nombreux et font généralement appel à la fluoration d'un précurseur chloré tel que le 2,2-difluoro-4,4,4-trichlorobutane, le 2-bromo-2,4,4,4-tétrachlorobutane et le 1,1,1,3,3-pentachlorobutane.

Henne et coll. (J. Am. Chem. Soc. 67, p. 1194-1197 et 1197-1199, 1945) chlorent le 2,2-difluorobutane pour obtenir avec une sélectivité de 52,5 % le 2,2-difluoro-4,4,4-trichlorobutane (F362 jfc) qui est ensuite fluoré en F365 mfc. McBee et Hausch (Ind. Eng. Chem. 39, p.418-420, 1947) fluorent le F362 jfc par HF/HgO ou par le mélange SbF₃/SbCl₅, mais les rendements de fluoration ne dépassent pas 15 %. Tous ces procédés se caractérisent par de faibles rendements dûs essentiellement au manque de sélectivité des réactions de chloration conduisant au précurseur chloré.

Une autre méthode de préparation du F365 mfc, décrite par Tarrant et Coll. (J. Am. Chem. Soc. 80, p.1711-1713, 1958) consiste en l'addition radicalaire de CCl₃Br sur le 2-chloropropène et la fluoration par HF, en l'absence de catalyseur, du produit d'addition 1:1 (CCl₃CH₂CBrClCH₃) obtenu avec un rendement de 34 %. Cette méthode n'améliore pas le rendement global puisque, dans ce cas, on observe un manque de sélectivité dû à la formation de télomères favorisée par l'initiation radicalaire au peroxyde de benzoyle.

Un autre précurseur chloré du F365 mfc est le 1,1,1,3,3-pentachlorobutane qui, selon Friedlina et Coll. [Izv. Akad. Nauk SSSR (6), p.1333-1336 (1980) et (8), p.1903-5 (1979)], peut être obtenu par télomérisation du chlorure de vinylidène par le 1,1,1-trichloroéthane ou celle du 2-chloropropène par CCl₄ en présence de fer pentacarbonyle. Dans les deux cas, il est obtenu un mélange de trois télomères et la sélectivité en 1,1,1,3,3-pentachlorobutane (produit d'addition 1:1) est insuffisante pour présenter un intérêt économique certain.

La préparation directe du F365 mfc selon la méthode de Bloshchitsa et Coll. (Zhur. Org. Khim 24(7), p. 1558, 1988) par action de l'acide fluorhydrique et du tétrafluorure de soufre sur le dicétène, constitue en fait la seule méthode conduisant au F365 mfc avec un rendement acceptable (70 %). Malheureusement, ce procédé fait appel à deux matières premières d'usage peu courant, le dicétène et le tétrafluorure de soufre.

Le F365 mfc est aussi un sous-produit de la fabrication du 1,1-dichloro-1-fluoroéthane (F141b), mais la proximité de leurs points d'ébullition (F141b: Eb. = 32°C ; F365 mfc : Eb. = 40°C) et l'existence d'un mélange azéotropique à point d'ébullition minimum ne permettent pas une séparation facile des deux produits. Un procédé de séparation, basé sur le principe d'une distillation en présence d'un excès d'HF, est cependant décrit dans le brevet EP 395 793.

Les complexes amine/sel cuivreux sont des catalyseurs connus pour l'addition de composés polyhalogénés sur les oléfines (Kotora et Coll., J. of Molecular Catalysis 77, p.51-61, 1992), mais ces auteurs montrent que le rendement en produit d'addition 1:1 peut passer de 97 % pour le chlorure de vinyle à 11 % pour le 1,2-dichloroéthylène.

On connaît également, par le brevet américain US 3 862 978, une méthode générale de réaction d'un composé insaturé avec un composé halogéné en présence d'un catalyseur comprenant du cuivre et une amine spécifique, ladite méthode permettant la préparation d'un produit d'addition 1:1 Ce document ne mentionne cependant pas spécifiquement le 2-chloropropène.

En recherchant un procédé permettant de préparer le F365 mfc avec un rendement et une sélectivité élevés, il a maintenant été trouvé que le 2-chloropropène se classe parmi les oléfines réactives vis-à-vis du tétrachlorure de carbone et peut, dans certaines conditions, conduire au produit d'addition 1:1 attendu, le 1,1,1,3,3-pentachlorobutane, avec un excellent rendement et une excellente sélectivité, la formation de sous-produits, télomères ou produits d'élimination étant négligeable. De plus, parmi ces sous-produits, les oléfines C₄H₄Cl₄ éventuellement formées par déshydrochloration du 1,1,1,3,3-pentachlorobutane se fluorent facilement en 1,1,1,3,3-pentafluorobutane.

L'invention a donc pour premier objet un procédé de préparation du 1,1,1,3,3-pentachlorobutane par addition du tétrachlorure de carbone sur le 2-chloropropène, caractérisé en ce que l'on opère en présence d'halogénure cuivreux, et d'une mono, di- ou trialkylamine dont la concentration molaire est comprise entre 2,5 et 6 %.

L'invention a également pour objet un procédé de préparation du F365 mfc comprenant une première étape d'addition du tétrachlorure de carbone sur le 2-chloropropène et une étape de fluoruration par l'acide fluorhydrique du 1,1,1,3,3-pentachlorobutane ainsi obtenu.

Comme halogénure cuivreux, on utilise avantageusement le chlorure cuivreux.

Le ou les radicaux alkyle de l'amine sont des radicaux alkyle, linéaire(s) ou ramifié(s), pouvant contenir de 1 à 8 (de préférence 2 à 4) atomes de carbone. On peut également utiliser une aminé cyclanique, par exemple la cyclohexylamine. On utilise avantageusement une amine primaire et, plus particulièrement, l'isopropylamine.

Le 2-chloropropène peut être obtenu de façon connue en soi, par déshydrochloration du 1,2-dichloropropane ou du 2,2-dichloropropane (thermiquement ou par action de la potasse glycolique) ou, de préférence, par action du phénylchloroforme sur l'acétone en présence d'un acide de Lewis comme le chlorure de zinc ou le chlorure ferrique (brevet FR 2 213 257); dans ce cas, le rendement en 2-chloropropène atteint 77 % et le principal sous-produit de la réaction, le 2,2-dichloropropane, peut aisément être converti en 2-chloropropène par élimination d'HCl selon le procédé à la potasse ou à la soude glycolique décrit dans le brevet US 2 543 648.

Le rapport molaire CCl₄/CH₃CCl = CH₂ peut aller de 2 à 6, mais il est de préférence compris entre 2,5 et 4,5.

L'halogénure cuivreux, en particulier la chlorure cuivreux, a pour rôle d'amorcer la formation du radical trichlorométhyle et d'assurer le transfert du chlore sur le radical CCl₃CH₂CClCH₃ résultant de l'addition du radical CCl₃ sur le 2-chloropropène. Il est généralement utilisé en une quantité telle que le rapport molaire : halogénure cuivreux/2-chloropropène soit compris entre 0,001 et 0,05, de préférence entre 0,005 et 0,02.

La concentration en mono, di- ou trialkylamine est rapportée au nombre de moles total du mélange réactionnel initial (CCl₄ + CH₃CCl = CH₂ + CuCI + amine).

La réaction peut être effectuée à une température comprise entre 80 et 130°C, mais on opère de préférence entre 90 et 110°C.

Le 1,1,1,3,3-pentachlorobutane formé qui peut être séparé du mélange réactionnel par des procédés connus en soi, notamment par filtration, lavage acide, lavage à l'eau, séchage et distillation, est utilisé conformément au second aspect de la présente invention pour la synthèse du 1,1,1,3,3-pentafluorobutane par fluoruration au moyen d'acide fluorhydrique.

Cette opération peut être réalisée en phase liquide, en présence ou en l'absence de catalyseur. Elle est généralement effectuée sous pression autogène à une température comprise entre 80 et 120°C, de préférence d'environ 100°C. Le rapport molaire HF/CCl₃CH₂CCl₂CH₃ peut aller de 15 à 30, mais il est avantageusement compris entre 20 et 25. Comme catalyseur, on peut utiliser n'importe quel catalyseur de fluoruration en phase liquide, en particulier un catalyseur à base d'antimoine. Les meilleurs résultats ont cependant été obtenus sans catalyseur.

Les exemples suivants illustrent l'invention sans la limiter.

### EXEMPLE 1

Dans un tube en Pyrex à paroi épaisse maintenu à 0°C, on a introduit successivement 8 mg de chlorure cuivreux, 117 mg de n.butylamine, 370 mg de 2-chloropropène (préalablement déstabilisé par distillation) et enfin 3,2g de tétrachlorure de carbone. Le tube a alors été scellé, homogénéisé et chauffé à température constante (100°C) durant 4 heures, sous agitation.

Après refroidissement, le tube a été ouvert sur une rampe à gaz permettant de récupérer quantitativement la phase gazeuse, la phase liquide étant transvasée dans un flacon pour une analyse séparée. Les deux phases ont été analysées par chromatographie en phase gazeuse et le bilan de la réaction reconstitué à partir de ces analyses.

Les résultats de cet essai (Essai 1-a), ainsi que les conditions opératoires et les résultats obtenus avec d'autres amines (Essais 1-b à 1-g), sont résumés dans le tableau (I) où les abréviations ont les significations suivantes:

| | |
|---|---|
| T.T. | taux de transformation |
| 360 jfa | 1,1,1,3,3-pentachlorobutane |

**TABLEAU I**

| **ESSAI N°** | **1-a** | **1-b** | **1-c** | **1-d comparatif** | **1-e** | **1-f** | **1-g** |
|---|---|---|---|---|---|---|---|
| **Amine** | **n.butylamine** | **isopropylamine** | **tertbutylamine** | **éthanolamine** | **diéthylamine** | **triéthylamine** | **cyclohexylamine** |
| ***Conditions opératoires*** : | | | | | | | |
| | | | | | | | |
| . concentration en amine (% molaire) | 5,9 | 5 | 3,35 | 1,24 | 4 | 4,7 | 6,0 |
| | | | | | | | |
| . rapport molaire CCl₄/ 2-chloropropène | 4,3 | 4 | 4 | 4 | 4 | 4,1 | 3,9 |
| | | | | | | | |
| . rapport molaire CuCl/2-chloropropène | 0,017 | 0,014 | 0,014 | 0,01 | 0,02 | 0,02 | 0,016 |
| ***Résultats*** : | | | | | | | |
| | | | | | | | |
| T.T. (%) du 2-chloropropène | 99,7 | 98,4 | 99,3 | 2,7 | 84,3 | 57,1 | 95,4 |
| | | | | | | | |
| Sélectivité (%) en : | | | | | | | |
| - 360 jfa | 90,7 | 94,2 | 85,7 | 0,8 | 68 | 37,1 | 90,3 |
| - oléfines | 1,8 | 0,3 | 3,8 | 0,4 | 0,2 | 1 | 1,3 |
| - lourds | 7,2 | 3,8 | 9,9 | 1,6 | 16,1 | 19 | 9,4 |
| | | | | | | | |
| T.T. (%) du CCl₄ en CHCl₃ | 1,8 | 1 | 0,3 | 1 | 0,7 | 0,7 | 1,1 |
| | | | | | | | |

### EXEMPLE 2

On a opéré comme à l'exemple 1 avec l'isopropylamine, mais en faisant varier sa concentration dans le mélange réactionnel.

Les résultats sont rassemblés dans le tableau Il suivant.

**TABLEAU II**

| **ESSAI N°** | **2-a** | **2-b** | **2-c(*)** | **1-b** | **2-d(**)** | **2-e** |
|---|---|---|---|---|---|---|
| ***Conditions opératoires* :** | | | | | | |
| | | | | | | |
| . concentration en isopropylamine (% molaire) | 0,5 | 1,0 | 2,6 | 5,0 | 6,9 | 7,5 |
| | | | | | | |
| . rapport molaire CCl₄/ 2-chloropropène | 4,4 | 4,2 | 4,1 | 4,0 | 4,1 | 3,9 |
| | | | | | | |
| . rapport molaire CuCl/2-chloropropène | 0,0136 | 0,0094 | 0,0197 | 0,014 | 0,0310 | 0,0130 |
| ***Résultats* :** | | | | | | |
| | | | | | | |
| T.T. du 2-chloropropène (%) | 1 | 54 | 97,8 | 98,4 | 79,3 | 57,4 |
| | | | | | | |
| Sélectivité (%) en : | | | | | | |
| - 360 jfa | 1 | 53,6 | 93,5 | 94,2 | 69,1 | 34 |
| - oléfines | 0 | 0 | 0,4 | 0,3 | 3,6 | 6,4 |
| - lourds | 0 | 0,4 | 4,0 | 3,8 | 6,4 | 17,2 |
| | | | | | | |
| T.T. du CCl₄ en CHCl₃ (%) | 0,2 | 0,5 | 0,6 | 1,0 | 2,1 | 1,3 |

| | | | | | | |
|---|---|---|---|---|---|---|
| (*) : 6 heures à 100°C au lieu de 4 heures à 100°C | | | | | | |
| (**) : 2 heures à 100°C au lieu de 4 heures à 100°C | | | | | | |

### EXEMPLE 3

Dans un ballon tricol de 500 ml équipé d'un condenseur à -25°C et d'une ampoule de coulée, on a introduit successivement 1g (0,01 mole) de chlorure cuivreux, 4,60g (0,077 mole) d'isopropylamine, puis après agitation, 180,5 g de CCl₄ (1,17 mole). Dans la solution colorée en bleu vif, on a introduit alors 29,6g (0,38 mole) de 2-chloropropène et on a chauffé le milieu réactionnel à 100°C durant deux heures et trente minutes.

Après arrêt du chauffage et refroidissement durant 6 heures, le mélange réactionnel a été filtré sur "Décalite" jusqu'à disparition de la coloration bleu-verte, puis lavé deux fois à l'acide chlorhydrique 1N pour éliminer les traces d'amine n'ayant pas réagi. La phase organique a ensuite été lavée à l'eau jusqu'à neutralité et séchée sur sulfate de sodium. Le 1,1,1,3,3-pentachlorobutane a ensuite été séparé de l'excès de CCl₄ par distillation sous vide entre la température ambiante et 50°C.

A côté de 1,4 g de 2-chloropropène n'ayant pas réagi (T.T. = 95,3 %), on a obtenu 79 g de 1,1,1,3,3-pentachlorobutane, 1,7 g d'oléfine C₄Cl₄H₄ et 2,7 g de télomères lourds dont 90 % de dimère. La pureté du 360 jfa ainsi obtenu par distillation atteint 98 % et le taux de transformation du 2-chloropropène en 360 jfa est de 88,6 %; si l'on considère que l'oléfine se fluore en F365 mfc de la même manière que le 360 jfa, le rendement global atteint dans ces conditions 90,8 %.

### EXEMPLE 4

Dans un autoclave en acier inoxydable 316L de 800 ml, équipé d'un indicateur de pression, d'une sonde thermométrique, d'un disque d'éclatement et d'un système d'agitation par barreau magnétique, ont été introduits successivement 41,1 g de 1,1,1,3,3-pentachlorobutane (0,178 mole) et 82,2 g d'acide fluorhydrique anhydre (4,108 moles).

Le réacteur étant chauffé à 100°C, la pression a augmenté graduellement pour atteindre 30,4 bars. Après 8 heures, le système réactionnel a été ramené à température ambiante et une pression résiduelle de 12,8 bars a été observée.

Les composés organiques légers (23,2 g) et les hydracides (96,8 g) ont été éliminés par dégazage puis balayage à l'hélium. Les produits lourds (2 g) ont été collectés après ouverture de l'autoclave.

Les analyses des différentes phases recueillies ont permis de calculer un taux de transformation du 1,1,1,3,3-pentachlorobutane et une sélectivité en 1,1,1,3,3-pentafluorobutane qui atteignent respectivement 98,1 % et 61 %.

Les autres produits de réaction étaient principalement des composés sous-fluorés : C₄H₅ClF₄ (sélectivité = 14,8 %), C₄H₅Cl₂F₃ (2 isomères ; sélectivité = 17,8 %) et C₄H₅Cl₃F₂ (sélectivité = 1 %). Ces composés peuvent être avantageusement recyclés au réacteur, pour être transformés en 1,1,1,3,3-pentafluorobutane.

## Revendications

1. Procédé de préparation du 1,1,1,3,3-pentachlorobutane par addition du tétrachlorure de carbone sur le 2-chloropropène, **caractérisé en ce que** l'on opère en présence d'halogénure cuivreux et d'une mono, di- ou trialkylamine ou une amine cyclanique dont la concentration molaire est comprise entre 2,5 et 6 %.

2. Procédé selon la revendication 1, dans lequel l'halogénure cuivreux est le chlorure cuivreux.

3. Procédé selon la revendication 1 ou 2, dans lequel l'amine est une amine primaire contenant de 1 à 8 atomes de carbone, de préférence une alkylamine primaire contenant 2 à 4 atomes de carbone.

4. Procédé selon l'une des revendications 1 à 3, dans lequel l'amine est l'isopropylamine.

5. Procédé selon l'une des revendications 1 à 4, dans lequel le rapport molaire CCl₄/CH₃CCl = CH₂ est compris entre 2 et 6, de préférence entre 2,5 et 4,5.

6. Procédé selon l'une des revendications 1 à 5, dans lequel le rapport molaire : halogénure cuivreux/2-chloropropène est compris entre 0,001 et 0,05, de préférence entre 0,005 et 0,02.

7. Procédé selon l'une des revendications 1 à 6, dans lequel on opère à une température comprise entre 80 et 130°C, de préférence entre 90 et 110°C.

8. Procédé de préparation du 1,1,1,3,3-pentafluorobutane **caractérisé en ce que** l'on soumet un 1,1,1,3,3-pentachlorobutane obtenu par un procédé selon l'une des revendications 1 à 7, à une fluoruration par l'acide fluorhydrique.

9. Procédé selon la revendication 8 dans lequel la fluoruration est effectuée en phase liquide à une température allant d'environ 80 à 120°C, de préférence d'environ 100°C.

10. Procédé selon la revendication 8 ou 9, dans lequel le rapport molaire HF/CCl₃CH₂CCl₂CH₃ est compris entre 15 et 30, de préférence entre 20 et 25.

## Claims

1. Process for the preparation of 1,1,1,3,3-pentachlorobutane by addition of carbon tetrachloride to 2-chloropropene, **characterized in that** the process is performed in the presence of a cuprous halide and a mono-, di- or trialkylamine or a cyclanic amine, with a molar concentration of between 2.5% and 6%.

2. Process according to Claim 1, in which the cuprous halide is cuprous chloride.

3. Process according to Claim 1 or 2, in which the amine is a primary amine containing from 1 to 8 carbon atoms, preferably a primary alkylamine containing 2 to 4 carbon atoms.

4. Process according to one of Claims 1 to 3, in which the amine is isopropylamine.

5. Process according to one of Claims 1 to 4, in which the CCl₄/CH₃CCl=CH₂ molar ratio is between 2 and 6, preferably between 2.5 and 4.5.

6. Process according to one of Claims 1 to 5, in which the molar ratio: cuprous halide/2-chloropropene is between 0.001 and 0.05, preferably between 0.005 and 0.02.

7. Process according to one of Claims 1 to 6, which process is performed at a temperature of between 80 and 130°C, preferably between 90 and 110°C.

8. Process for the preparation of 1,1,1,3,3-pentafluorobutane, **characterized in that** a 1,1,1,3,3-pentachlorobutane obtained by a process according to one of Claims 1 to 7 is subjected to fluorination with hydrofluoric acid.

9. Process according to Claim 8, in which the fluorination is carried out in the liquid phase at a temperature ranging approximately from 80 to 120°C, preferably approximately 100°C.

10. Process according to Claim 8 or 9, in which the HF/CCl₃CH₂CCl₂CH₃ molar ratio is between 15 and 30, preferably between 20 and 25.

## Patentansprüche

1. Verfahren zur Herstellung von 1,1,1,3,3-Pentachlorbutan durch Addition von Kohlenstofftetrachlorid an 2-Chlorpropen,
**dadurch gekennzeichnet,**
**daß** man in Gegenwart von Kupfer(I)halogenid und eines Mono-, Di- oder Trialkylamins oder eines Cycloalkylamins verfährt, dessen molare Konzentration zwischen 2,5 und 6 % liegt.

2. Verfahren nach Anspruch 1, wobei das Kupfer(I)halogenid Kupfer(I)chlorid ist.

3. Verfahren nach Anspruch 1 oder 2, wobei das Amin ein primäres Amin mit 1 bis 8 Kohlenstoffatomen, vorzugsweise ein primäres Alkylamin mit 2 bis 4 Kohlenstoffatomen, ist.

4. Verfahren nach einem der Ansprüche 1 bis 3, wobei das Amin Isopropylamin ist.

5. Verfahren nach einem der Ansprüche 1 bis 4, wobei das Molverhältnis CCl₄/CH₃CCl=CH₂ zwischen 2 und 6, vorzugsweise zwischen 2,5 und 4,5, liegt.

6. Verfahren nach einem der Ansprüche 1 bis 5, wobei das Molverhältnis von Kupfer(I)halogenid/2-Chlorpropen zwischen 0,001 und 0,05, vorzugsweise zwischen 0,005 und 0,02, liegt.

7. Verfahren nach einem der Ansprüche 1 bis 6, wobei man bei einer Temperatur zwischen 80 und 130 °C, vorzugsweise zwischen 90 und 110 °C, verfährt.

8. Verfahren zur Herstellung von 1,1,1,3,3-Pentafluorbutan, **dadurch gekennzeichnet, daß** man ein 1,1,1,3,3-Pentachlorbutan, das gemäß einem Verfahren nach einem der Ansprüche 1 bis 7 erhalten worden ist, einer Fluorierung mittels Fluorwasserstoff unterwirft.

9. Verfahren nach Anspruch 8, wobei die Fluorierung in flüssiger Phase bei einer Temperatur von etwa 80 bis 120 °C, vorzugsweise etwa 100 °C, durchgeführt wird.

10. Verfahren nach Anspruch 8 oder 9, wobei das Molverhältnis von HF/CCl₃CH₂CCl₂CH₃ zwischen 15 und 30, vorzugsweise zwischen 20 und 25, liegt.
